# EUROPÄISCHE PATENTANMELDUNG

(11) **EP 4 064 291 A1**
(43) Veröffentlichungstag der Anmeldung: **28.09.2022**
(21) Anmeldenummer: 21163878.8
(22) Anmeldetag: 22.03.2021
(51) Int. Cl.: G16H 40/40

(54) **VERFAHREN ZUR UNTERSTUETZUNG EINES BENUTZERS BEI EINER EINSTELLUNG EINES PARAMETERWERTS VON ZUMINDEST EINEM MESSPARAMETER EINER MAGNETRESONANZSEQUENZ FÜR EINE MAGNETRESONANZUNTERSUCHUNG SOWIE EINE ENTSPRECHENDE MAGNETRESONANZVORRICHTUNG**

(71) Anmelder: Siemens Healthcare GmbH, 91052 Erlangen (DE)
(72) Erfinder: Kröll, Maria, 91052 Erlangen (DE)

(57) **Zusammenfassung**

Die Erfindung geht aus von einem Verfahren zu einem Unterstützen eines Benutzers bei einer Einstellung eines Parameterwerts von zumindest einem Messparameter einer Magnetresonanzsequenz für eine Magnetresonanzuntersuchung, umfassend:
- Manuelle Auswahl zumindest eines Messparameters und Eingabe eines gewünschten Parameterwerts für den zumindest einen Messparameter,
- Überprüfen des eingegebenen Parameterwerts des zumindest einen Messparameters, ob der eingegebene Parameterwert des zumindest einen Messparameters mit Einstellungen von Parameterwerten von weiteren Messparametern der Magnetresonanzsequenz inkompatibel ist,
- sofern der eingegebene Parameterwert des zumindest einen Messparameters mit den Einstellungen von Parameterwerten zumindest eines weiteren Messparameters der Magnetresonanzsequenz inkompatibel ist, Bestimmen von zumindest einer Option für eine Änderung eines Parameterwerts zumindest eines weiteren Messparameters, wobei die Änderung des Parameterwerts des zumindest einen weiteren Messparameters zu einer Kompatibilität des eingegebenen Parameterwerts des zumindest einen Messparameters mit den Einstellungen der Parameterwerte der weiteren Messparameter führt, und
- Darstellen der zumindest einen Option.

## Beschreibung

Die vorliegende Erfindung betrifft ein Verfahren zu einem Unterstützen eines Benutzers bei einer Einstellung eines Parameterwerts von zumindest einem Messparameter einer Magnetresonanzsequenz für eine Magnetresonanzuntersuchung. Des Weiteren betrifft die vorliegende Erfindung auch eine Magnetresonanzvorrichtung mit einer Sequenzsteuereinheit und einer Benutzerschnittstelle, wobei die Magnetresonanzvorrichtung zu einem Ausführen eines Verfahrens zu einem Unterstützen eines Benutzers bei einer Einstellung eines Parameterwerts von zumindest einem Messparameter einer Magnetresonanzsequenz für eine Magnetresonanzuntersuchung ausgebildet ist. Die Erfindung geht des Weiteren aus von einem Computerprogrammprodukt zu einem Ausführen des erfindungsgemäßen Verfahrens zu einem Unterstützen eines Benutzers bei einer Einstellung eines Parameterwerts von zumindest einem Messparameter einer Magnetresonanzsequenz für eine Magnetresonanzuntersuchung, sowie einen entsprechenden elektronisch lesbarer Datenträger, der das Computerprogrammprodukt umfasst.

Bevor eine Magnetresonanzuntersuchung an einem Patienten durchgeführt wird, werden häufig von einem Benutzer, insbesondere einem medizinischen Bedienpersonal, Parameterwerte einzelner Messparameter einer Magnetresonanzsequenz und/oder eines Magnetresonanzprotokolls an eine klinische und/oder medizinische Fragestellung angepasst. Jedoch besteht die Schwierigkeit bei einem Einstellen und/oder Anpassen der Parameterwerte einzelner Messparameter, dass diese nicht unabhängig von den weiteren Messparametern geändert werden können sondern Abhängigkeiten zwischen den einzelnen Messparametern vorhanden sind. Des Weiteren können bei einem Anpassen und/oder Einstellen der Parameterwerte einzelner Messparameter auch Auswirkungen auf die Magnetresonanzuntersuchung ergeben, wie beispielsweise eine Beeinflussung der Messzeit und/oder eine Beeinflussung einer Auflösung und/oder eines S/N-Verhältnisses. Dabei ist es für den Benutzer oft schwierig, diese Abhängigkeiten zu erkennen. Zudem ist es für den Benutzer schwierig zu erkennen, welche Parameter er überhaupt vorteilhaft verändern könnte, um eine gewünschte Anpassung, wie beispielsweise mehr Schichten für ein größeres Field of View (FOV), überhaupt zu ermöglichen. Weiterhin ist es für den Benutzer ebenfalls schwierig zu erkennen, welche Auswirkungen eine Änderung eines Parameterwerts eines Messparameters auf die Magnetresonanzmessung aufweist, wie beispielsweise Anpassungen weiterer Messparameter und/oder einer längeren Messzeit und/oder ein kleineres Field of View (FOV) usw.

Diese Auswirkungen sind für einen Benutzer oft erst ersichtlich, wenn er die Änderung eines Parameterwerts eines Messparameters festgelegt hat. Somit muss ein Benutzer häufig mehrere Versuche zur Änderung eines Messparameters tätigen, um die gewünschten Einstellungen sowohl in dem zu ändernden Messparameter als auch in den weiteren Messparametern zu erzielen.

Der vorliegenden Erfindung liegt insbesondere die Aufgabe zugrunde, eine vorteilhafte Unterstützung eines Benutzers bei einer Einstellung eines Parameterwerts eines Messparameters zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Unterstützen eines Benutzers bei einer Einstellung eines Parameterwerts von zumindest einem Messparameter einer Magnetresonanzsequenz für eine Magnetresonanzuntersuchung, umfassend die folgenden Verfahrensschritte:
- Manuelle Auswahl zumindest eines Messparameters und Eingabe eines gewünschten Parameterwerts für den zumindest einen Messparameter,
- Überprüfen des eingegebenen Parameterwerts des zumindest einen Messparameters, ob der eingegebene Parameterwert des zumindest einen Messparameters mit Einstellungen von Parameterwerten von weiteren Messparametern der Magnetresonanzsequenz kompatibel ist,
- Sofern der eingegebene Parameterwert des zumindest einen Messparameters mit den Einstellungen von Parameterwerten zumindest eines weiteren Messparameters der Magnetresonanzsequenz inkompatibel ist, Bestimmen von zumindest einer Option für eine Änderung eines Parameterwerts zumindest eines weiteren Messparameters, wobei die Änderung des Parameterwerts des zumindest einen weiteren Messparameters zu einer Kompatibilität des eingegebenen Parameterwerts des zumindest einen Messparameters mit den weiteren Messparametern führt, und
- Darstellen der zumindest einen Option.

Mittels einer Magnetresonanzuntersuchung an einem Patienten, insbesondere an einem zu untersuchenden Bereich des Patienten, soll eine klinische und/oder medizinische Fragestellung geklärt werden. Hierzu werden klinische und/oder medizinische Bilddaten, insbesondere klinische und/oder medizinische Magnetresonanzbilddaten, während der Magnetresonanzuntersuchung von dem zu untersuchenden Bereich des Patienten erfasst. Hierbei beschreibt eine Ablaufplan einer Magnetresonanzuntersuchung insbesondere einen Workflow, wobei der Ablaufplan und/oder Workflow vorzugsweise eine Reihenfolge von einzelnen Magnetresonanzprotokollen bei einer Ausführung der Magnetresonanzuntersuchung festlegt.

Ein Magnetresonanzprotokoll umfasst einen Satz an Werten, der benötigt wird, um eine konkrete Ausprägung einer Magnetresonanzsequenz zu beschreiben. Typische Werte können hierbei eine Echozeit (TE), eine Repetitionszeit (TR), ein Field of View, eine Matrix-Größe, eine Anzahl an Schichten, eine Fettsättigungsmethode, eine Beschleunigungsmethode, eine lokale Hochfrequenzantenneneinheit usw. umfassen. Häufig sind derartige Werte eines Magnetresonanzprotokolls auch stark von der vor Ort verfügbaren Hardware und/oder Ausgestaltung der Magnetresonanzvorrichtung abhängig, wie beispielsweise von einer Gradientenstärke der Magnetresonanzvorrichtung und/oder einer Anzahl an Aufnahmekanälen usw. Ein Magnetresonanzprotokoll beschreibt damit eine bestimmte Art und Weise, ein Bild mit einer ganz bestimmten Ausgestaltung einer Magnetresonanzvorrichtung aufzunehmen. Insbesondere sind dabei ein Kontrast und/oder eine Geometrie und eine Messdauer auf die klinische Fragestellung der anstehenden Magnetresonanzuntersuchung abgestimmt.

Eine Magnetresonanzsequenz für eine Magnetresonanzuntersuchung umfasst insbesondere eine logische Abfolge von Hochfrequenz-Pulsen (HF-Pulsen), Gradientenpulsen und Aufnahme-Zeiträumen zur Steuerung der Datenaufnahme für eine Magnetresonanzdatenerfassung. Die Magnetresonanzsequenz legt dabei die grundsätzlichen Magnetresonanzmechanismen fest, die bei der Magnetresonanzdatenerfassung und/oder einer Magnetresonanzdatenakquisition benutzt werden, wie beispielsweise ein Gradienten-Echo oder Spin-Echo, ein Steady-State der Magnetisierung, Präparationspulse usw. Typische und bekannte Magnetresonanzsequenz sind beispielsweise eine Spin-Echo-Sequenz (SE-Sequenz), eine Turbo-Spin-Echo-Sequenz (TSE-Sequenz), eine Echo Planar Imaging (EPI) usw. Eine Magnetresonanzsequenz legt jedoch nicht die vollständige zeitliche Ausprägung aller HF-Pulse, Gradientenpulse und Aufnahme-Zeiträume fest, sondern nur deren Zusammenspiel. Grundsätzlich kann eine Parametrisierung einer Magnetresonanzsequenz angepasst werden, beispielweise durch einen Benutzer und/oder ein medizinisches Bedienpersonal. Hierbei kann insbesondere ein Timing der Magnetresonanzsequenz und/oder eine Auflösung der Magnetresonanzsequenz an die klinische und/oder medizinische Fragestellung angepasst werden.

Die einzelnen Messparameter einer Magnetresonanzsequenz sind bereits mit einem Parameterwert vorbelegt und/oder voreingestellt, wobei die einzelnen Parameterwerte der Messparameter aufeinander abgestimmt und/oder optimiert sind. Für eine Anpassung einer Magnetresonanzsequenz an die klinische und/oder diagnostische Fragestellung kann es jedoch von Vorteil sein, einzelne Messparameter, insbesondere den Parameterwert einzelner Messparameter, anzupassen. Eine Auswahl eines Parameterwerts für einen Messparameter erfolgt bevorzugt aus einem zulässigen Wertebereich für diese Messparameter. Ein Wertebereich für einen Messparameter kann dabei einen kontinuierlichen Bereich, innerhalb dessen der Parameterwert zum Einstellen des Messparameters ausgewählt werden kann, umfassen. Alternativ hierzu kann der Wertebereich für einen Messparameter auch zumindest zwei Zustände umfassen, wie beispielsweise "an" oder "aus", wobei der Parameterwert einen dieser zumindest zwei Zustände annehmen kann.

Die manuelle Auswahl zumindest eines Messparameters erfolgt bevorzugt durch den Benutzer, insbesondere ein medizinisches Bedienpersonal, an einer Benutzerschnittstelle einer Magnetresonanzvorrichtung. Die Benutzerschnittstelle weist hierzu insbesondere eine Eingabeeinheit, wie beispielsweise eine Tastatur und/oder eine Computermaus usw., und eine Ausgabeeinheit und/oder Darstellungseinheit, wie beispielsweise ein Monitor und/oder ein Display, auf. Zudem kann die Eingabeeinheit auch einteilig und/oder einstückig mit der Ausgabeeinheit ausgebildet sein, wie beispielsweise bei einer Ausbildung als Touch-Display. Mittels der Benutzerschnittstelle kann der Benutzer zumindest einen Messparameter auswählen und für diesen eine gewünschten Parameterwert eingeben. Der Messparameter kann beispielsweise eine Anzahl an Schichten und/oder ein Field of View (FOV) und/oder eine Auflösung und/oder eine Schichtdicke usw. umfassen, wobei der Benutzer für diesen zumindest einen Messparameter den Parameterwert eine gewünschte Anpassung und/oder eine gewünschte Änderung eingeben kann. Der gewünschte Parameterwert des zumindest einen Messparameters umfasst somit den Parameterwert, den der Benutzer bei der anstehenden Magnetresonanzuntersuchung verwenden möchte.

Bevor der gewünschte Parameterwert bzw. der eingegebene Parameterwert für die anstehende Magnetresonanzuntersuchung bestätigt und/oder festgelegt wird, erfolgt in einem weiteren Verfahrensschritt ein Überprüfen des eingegebenen und/oder gewünschten Parameterwerts des zumindest einen Messparameters. Die Überprüfung erfolgt bevorzugt mittels einer Sequenzsteuereinheit der Magnetresonanzvorrichtung, insbesondere erfolgt die Überprüfung automatisch und/oder selbsttätig mittels der Sequenzsteuereinheit. Die Überprüfung des eingegebenen Parameterwerts für den zumindest einen Messparameter umfasst eine Inkompatibilitätsprüfung, ob der eingegebene Parameterwert und/oder der gewünschte Parameterwert des zumindest einen Messparameters mit Einstellung von Parameterwerten von weiteren Messparametern der Magnetresonanzsequenz inkompatibel ist. Eine Inkompatibilität zwischen den einzelnen Messparametern, insbesondere den Parameterwerten der einzelnen Messparameter, liegt vor, wenn der eingegebene Parameterwert des zumindest einen Messparameters für eine Ausführung der Magnetresonanzsequenz zu einer Änderung in Parameterwerten von zumindest einem weiteren Messparameter führt. Die Einstellungen von Parameterwerten der weiteren Messparameter sind bereits voreingestellt und für eine Ausführung der Messsequenz aufeinander abgestimmt und/oder optimiert.

Sofern eine Inkompatibilität zwischen dem eingegebenen Parameterwert des zumindest einen Messparameters und den Einstellungen von Parameterwerten zumindest eines weiteren Messparameters vorliegt, erfolgt in einem weiteren Verfahrensschritt ein Bestimmen von zumindest einer Option für eine Änderung eines Parameterwerts zumindest eines weiteren Messparameters. Das Bestimmen der Option für eine Änderung des Parameterwerts des zumindest einen weiteren Messparameters wird mittels der Sequenzsteuereinheit ausgeführt, insbesondere automatisch und/oder selbsttätig mittels der Sequenzsteuereinheit ausgeführt. Die Option für die Änderung des Parameterwerts des zumindest einen weiteren Messparameters führt zu einer Kompatibilität des eingegebenen Parameterwerts des zumindest einen Messparameters mit den Einstellungen der Parameterwerte der weiteren Messparameter. Eine Option umfasst in diesem Zusammenhang einen Vorschlag zum Einstellen der Parameterwerte der Messparameter. Insbesondere umfasst eine Option einen Vorschlag zum Einstellen des Parameterwerts des zumindest einen ausgewählten Messparameters mit den eingegebenen und/oder gewünschten Parameterwert, wobei der Vorschlag zudem eine weitere Änderung und/oder Anpassung eines Parameterwerts zumindest eines weiteren Messparameters umfasst.

Beispielsweise kann der voreingestellte Parameterwert für eine Anzahl an Schichten für eine bestimmte Magnetresonanzsequenz 10 betragen. Bei der Anpassung der Magnetresonanzsequenz an den Patienten und/oder an die klinische und/oder diagnostische Fragestellung kann ein gewünschter Parameterwert für die Anzahl an Schichten erhöht werden, beispielsweise auf den Wert 30. Diese Änderung, insbesondere Erhöhung, in der Anzahl der Schichten kann dabei zu einer Änderung eines Parameterwerts eines weiteren Messparameters führen. Beispielsweise kann eine Option für eine Änderung eines Parameterwerts zumindest eines weiteren Messparameters eine Erhöhung einer Echozeit (TE) umfassen, wobei die Erhöhung der Echozeit mit der Erhöhung der Anzahl der Schichten kompatibel ist. Ein weiteres Beispiel für eine Option einer Änderung eines Parameterwerts zumindest eines weiteren Messparameters kann eine Reduzierung eines FOVs umfassen, wobei die Reduzierung des FOVs mit der Erhöhung der Anzahl der Schichten kompatibel ist. Die unterschiedlichen Optionen können zudem unabhängig voneinander sein, so dass jede Option für sich mit der gewünschten Änderung des Parameterwerts des zumindest einen Messparameters kompatibel ist.

Das Darstellen der zumindest einen Option erfolgt mittels einer Darstellungseinheit und/oder Anzeigeeinheit der Benutzerschnittstelle. Hierzu wird von der Sequenzsteuereinheit eine Ausgabeinformation umfassend die zumindest eine Option generiert und für eine Darstellung bereitgestellt.

Die Sequenzsteuereinheit umfasst zumindest ein Rechenmodul und/oder einen Prozessor, wobei die Sequenzsteuereinheit zum Ausführen von einzelnen Schritten des Verfahrens zu einem Unterstützen eines Benutzers bei einer Einstellung eines Parameterwerts von zumindest einem Messparameter einer Magnetresonanzsequenz für eine Magnetresonanzuntersuchung ausgebildet ist. So ist insbesondere die Sequenzsteuereinheit dazu ausgebildet, computerlesbare Instruktionen auszuführen, um einzelne Schritte des erfindungsgemäßen Verfahrens auszuführen. Insbesondere umfasst die Sequenzsteuereinheit eine Speichereinheit, wobei auf der Speichereinheit computerlesbare Informationen gespeichert sind, wobei die Sequenzsteuereinheit dazu ausgebildet ist, die computerlesbaren Informationen von der Speichereinheit zu laden und die computerlesbaren Informationen auszuführen, um einzelne Schritte des erfindungsgemäßen Verfahrens zu einem Unterstützen eines Benutzers bei einer Einstellung eines Parameterwerts von zumindest einem Messparameter einer Magnetresonanzsequenz für eine Magnetresonanzuntersuchung auszuführen.

Die Komponenten der Sequenzsteuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Magnetresonanzuntersuchung wird mittels einer Magnetresonanzvorrichtung, insbesondere einer medizinischen und/oder diagnostischen Magnetresonanzvorrichtung, durchgeführt. Die Magnetresonanzvorrichtung ist zu einem Erfassen von medizinischen und/oder diagnostischen Bilddaten, insbesondere medizinischen und/oder diagnostischen Magnetresonanzbilddaten, eines Patienten ausgelegt und/oder ausgebildet. Die Magnetresonanzvorrichtung umfasst hierzu eine Scannereinheit. Die Scannereinheit der Magnetresonanzvorrichtung umfasst bevorzugt eine Magneteinheit zur Erfassung der medizinischen und/oder diagnostischen Magnetresonanzbilddaten. Bevorzugt umfasst hierbei die Scannereinheit, insbesondere die Magneteinheit, einen Grundmagneten, ein Gradienten-System und eine Hochfrequenzantenneneinheit. Die Hochfrequenzantenneneinheit ist fest innerhalb der Scannereinheit angeordnet und zur Aussendung eine Anregungspulses ausgelegt und/oder ausgebildet. Des Weiteren weist die Magnetresonanzvorrichtung zumindest eine lokalen Hochfrequenzspule auf, die zu einem Empfangen eines Magnetresonanzsignals ausgebildet ist. Hierzu wird die lokale Hochfrequenzspule um den zu untersuchenden Bereich des Patienten angeordnet und/oder angelegt. Bevorzugt sind einzelne lokale Hochfrequenzspulen speziell auf einen Untersuchungsbereich der Patienten ausgelegt, wie beispielsweise eine Kopfhochfrequenzspule oder eine Kniehochfrequenzspule usw.

Der Grundmagnet ist zur Erzeugung eines homogenen Grundmagnetfelds mit einer definierten und/oder bestimmten Magnetfeldstärke, wie beispielsweise mit einer definierten und/oder bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Das Gradienten-System ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt und/oder ausgebildet. Beispielsweise ist hierzu der Patientenaufnahmebereich zylinderförmig ausgebildet und/oder zylinderförmig von der Scannereinheit, insbesondere der Magneteinheit, umgeben. Innerhalb des Patientenaufnahmebereichs ist bevorzugt das FOV und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Durch die Erfindung kann der Benutzer, insbesondere das medizinische Bedienpersonal, vorteilhaft bei einer Einstellung und/oder Anpassung von Parameterwerten von einzelnen Messparametern unterstützt werden. Insbesondere erhält derart der Benutzer einen schnellen Überblick über die mit seiner gewünschten Änderung eines Parameterwerts des zumindest einen Messparameters verbundenen Änderungen in weiteren Messparametern. Hierdurch kann der Benutzer vorteilhaft bei einer Entscheidung und/oder einer Auswahl einer Option unterstützt werden, da alle mit einer Option verknüpften Änderungen und/oder Anpassungen in den Einstellungen für den Benutzer als eine Art Vorschau dargestellt werden. Insbesondere kann der Benutzer derart die einzelnen Optionen vorteilhaft mit der medizinischen und/oder klinischen Fragestellung abgleichen.

In einer vorteilhaften Weiterbildung des erfindungsgemäßen Verfahrens kann es vorgesehen sein, dass die zumindest eine Option eine Auswirkung der Änderungen des Parameterwerts des zumindest einen weiteren Messparameters auf die Magnetresonanzmessung umfasst. Vorzugsweise wird dabei die zumindest eine Option zusammen mit der Auswirkung auf die Magnetresonanzvorrichtung vor einer Bestätigung des gewählten Messparameters und/oder vor einer Auswahl einer Option angezeigt. Somit stellt die zumindest eine Option eine Art Vorschau dar, die aufzeigt, welchen Einfluss die zumindest eine Option auf die Magnetresonanzmessung bei einer Ausführung der zumindest einen Option hat. Vorzugsweise werden die einzelnen Optionen zusammen mit den jeweiligen Auswirkungen, die diese bei einer Einstellung der Parameterwerte gemäß der Option auf die Magnetresonanzuntersuchung haben würde, für einen Benutzer dargestellt. Beispielsweise kann eine Option für eine Änderung eines Parameterwerts zumindest eines weiteren Messparameters eine Erhöhung einer Echozeit (TE) umfassen, die als Auswirkung in einer Erhöhung der Messzeit für die Magnetresonanzuntersuchung resultiert. Ein weiteres Beispiel für eine Option einer Änderung eines Parameterwerts zumindest eines weiteren Messparameters kann eine Reduzierung eines Field of Views (FOV) umfassen, wobei die Auswirkung eine Reduzierung eines S/N-Verhältnisses (Signal to Noise ratio) umfassen kann. Diese Ausgestaltung der Erfindung weist den Vorteil auf, dass ein Benutzer eine Auswahl einer Option in Abhängigkeit von einer Messstrategie, wie beispielsweise eine zeitsparenden Magnetresonanzmessung und/oder eine besonders geräuscharme Magnetresonanzmessung usw., und/oder einer medizinischen Fragestellung und/oder einem Zustand des Patienten, beispielsweise sollte bei einem bereits unruhigen und/oder nervösen Patienten dieser keiner zusätzlichen Messzeit ausgesetzt werden usw., auswählen kann.

In einer vorteilhaften Weiterbildung des erfindungsgemäßen Verfahrens kann es vorgesehen sein, dass zwei oder mehr Optionen bestimmt und angezeigt werden. Die zwei oder mehr Optionen umfassen bevorzugt zwei verschiedene Alternativen für eine Einstellung des Parameterwerts des zumindest einen Messparameters mit dem gewünschten und/oder eingegebenen Parameterwert für den zumindest einen Messparameter. Vorteilhafterweise werden die zwei oder mehr Optionen gleichzeitig für den Benutzer dargestellt. Diese Ausgestaltung der Erfindung weist den Vorteil auf, dass ein Benutzer eine schnellen Überblick über mehrere Optionen und/oder Vorschläge zur Einstellung des gewünschten Parameterwerts des zumindest einen Messparameters erhält. Aufgrund des Überblicks über zwei oder mehr Optionen und/oder Vorschläge kann zudem der Benutzer auch die Auswahl einer Option vorteilhaft im Hinblick auf die medizinische und/oder diagnostische Fragestellung abstimmen.

In einer vorteilhaften Weiterbildung des erfindungsgemäßen Verfahrens kann es vorgesehen sein, dass eine Option der zwei oder mehr Optionen ein Beibehalten der voreingestellten Parameterwerte aller Messparameter umfasst. Das Beibehalten der voreingestellten Parameterwerte aller Messparameter umfasst bevorzugt das Beibehalten der voreingestellten Parameterwerte für die weiteren Messparameter und auch das Beibehalten des voreingestellten Parameterwerts für den zumindest einen ausgewählten Messparameter. Hierdurch erhält ein Benutzer die Möglichkeit, alle Änderungen und/oder Anpassungen der Parameterwerte abzulehnen, wenn z.B. die Änderungen in den Parameterwerten unerwünschte Auswirkungen auf die Magnetresonanzuntersuchung aufweisen.

In einer vorteilhaften Weiterbildung des erfindungsgemäßen Verfahrens kann es vorgesehen sein, dass eine Option ausgewählt wird und die Parameterwerte der Messparameter gemäß der ausgewählten Option eingestellt werden. Vorzugsweise erfolgt die Auswahl der einen Option manuell durch den Benutzer, insbesondere mittels einer Benutzerschnittstelle der Magnetresonanzvorrichtung. Das Einstellen der einen ausgewählten Option erfolgt mittels der Sequenzsteuereinheit, insbesondere automatisch und/oder selbsttätig mittels der Sequenzsteuereinheit, der Magnetresonanzvorrichtung. Derart können die Parameterwerte besonders schnell eingestellt und/oder angepasst werden und damit eine Aufwand für ein medizinisches Bedienpersonal reduziert werden.

Des Weiteren geht die Erfindung aus von einer Magnetresonanzvorrichtung mit einer Sequenzsteuereinheit und einer Benutzerschnittstelle, wobei die Magnetresonanzvorrichtung zu einem Ausführen eines Verfahrens zu einem Unterstützen eines Benutzers bei einer Einstellung eines Parameterwerts von zumindest einem Messparameter einer Magnetresonanzsequenz für eine Magnetresonanzuntersuchung ausgebildet ist.

Durch die erfindungsgemäße Magnetresonanzvorrichtung kann der Benutzer, insbesondere das medizinische Bedienpersonal, vorteilhaft bei einer Einstellung und/oder Anpassung von Parameterwerten von einzelnen Messparametern unterstützt werden. Insbesondere erhält derart der Benutzer einen schnellen Überblick über die mit seiner gewünschten Änderung eines Parameterwerts des zumindest einen Messparameters verbundenen Änderungen in weiteren Messparametern. Hierdurch kann der Benutzer vorteilhaft bei einer Entscheidung und/oder einer Auswahl einer Option unterstützt werden, da alle mit einer Option verknüpften Änderungen und/oder Anpassungen in den Einstellungen für den Benutzer als eine Art Vorschau dargestellt werden. Insbesondere kann der Benutzer derart die einzelnen Optionen vorteilhaft mit der medizinischen und/oder klinischen Fragestellung abgleichen.

Die Vorteile der erfindungsgemäßen Magnetresonanzvorrichtung entsprechen im Wesentlichen den Vorteilen des erfindungsgemäßen Verfahrens zu einem Unterstützen eines Benutzers bei einer Einstellung eines Parameterwerts von zumindest einem Messparameter einer Magnetresonanzsequenz für eine Magnetresonanzuntersuchung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Steuereinheit ladbar ist, mit Programmmitteln, um ein Verfahren zu einem Unterstützen eines Benutzers bei einer Einstellung eines Parameterwerts von zumindest einem Messparameter einer Magnetresonanzsequenz für eine Magnetresonanzuntersuchung auszuführen, wenn das Programm in der Steuereinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit und/oder Steuereinheit ladbar und weist Programmcode-Mittel auf, um ein erfindungsgemäßes Verfahren zu einem Unterstützen eines Benutzers bei einer Einstellung eines Parameterwerts von zumindest einem Messparameter einer Magnetresonanzsequenz für eine Magnetresonanzuntersuchung auszuführen, wenn das Computerprogrammprodukt in der Recheneinheit und/oder Steuereinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit und/oder Steuereinheit die erfindungsgemäßen Verfahrensschritte ausführen kann. Die Recheneinheit und/oder Steuereinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit und/oder Steuereinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Recheneinheit und/oder Steuereinheit gespeichert werden, können alle erfindungsgemäßen Ausführungsformen der vorab beschriebenen Verfahrens durchgeführt werden. So kann die Erfindung auch von dem besagten computerlesbaren Medium und/oder dem besagten elektronisch lesbaren Datenträger ausgehen.

Des Weiteren geht die Erfindung aus von einem computerlesbaren Datenträger, welcher ein Programm umfasst, das zu einer Ausführung eines Verfahrens zu einem Unterstützen eines Benutzers bei einer Einstellung eines Parameterwerts von zumindest einem Messparameter einer Magnetresonanzsequenz für eine Magnetresonanzuntersuchung vorgesehen ist.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanzvorrichtung in einer schematischen Darstellung und
- Fig. 2: ein Ablaufplan eines erfindungsgemäßen Verfahrens zu einem Unterstützen eines Benutzers bei einer Einstellung eines Parameterwerts von zumindest einem Messparameter einer Magnetresonanzsequenz für eine Magnetresonanzuntersuchung.

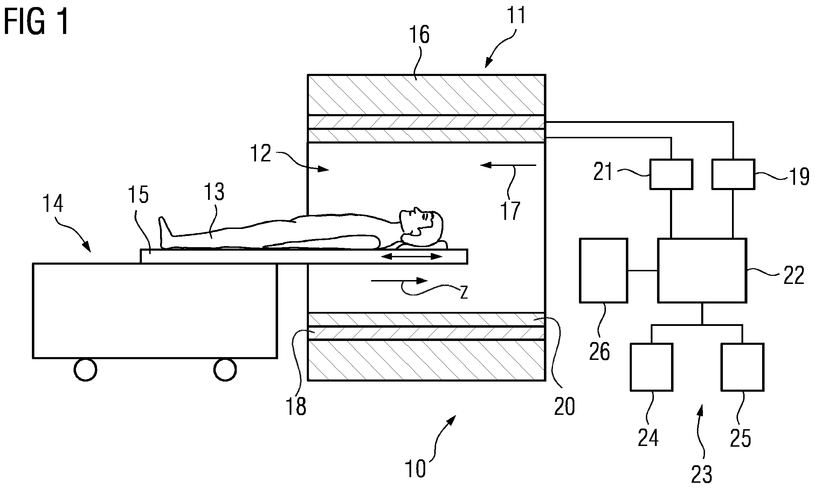

In der Fig. 1 ist eine Magnetresonanzvorrichtung 10 schematisch darstellt. Die Magnetresonanzvorrichtung 10 umfasst eine von einer Magneteinheit gebildeten Scannereinheit 11. Zudem weist die Magnetresonanzvorrichtung 10 einen Patientenaufnahmebereich 12 auf zu einer Aufnahme eines Patienten 13. Der Patientenaufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Patientenaufnahmebereichs 12 jederzeit denkbar. Der Patient 13 kann mittels einer Patientenlagerungsvorrichtung 14 der Magnetresonanzvorrichtung 10 in den Patientenaufnahmebereich 12 geschoben und/oder gefahren werden. Die Patientenlagerungsvorrichtung 14 weist hierzu einen innerhalb des Patientenaufnahmebereichs 12 bewegbar ausgestalteten Patiententisch 15 auf. Insbesondere ist hierbei der Patiententisch 15 in Richtung einer Längserstreckung des Patientenaufnahmebereichs 12 und/oder in z-Richtung bewegbar gelagert.

Die Scannereinheit 11, insbesondere die Magneteinheit, umfasst einen supraleitenden Grundmagneten 16 zu einem Erzeugen eines starken und insbesondere konstanten Grundmagnetfelds 17. Weiterhin weist die Scannereinheit 11, insbesondere die Magneteinheit, eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11, insbesondere die Magneteinheit, umfasst weiterhin eine Hochfrequenzantenneneinheit 20 zu einer Anregung einer Polarisation, die sich in dem von dem Grundmagneten 16 erzeugten Grundmagnetfeld 17 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in den Patientenaufnahmebereich 12 der Magnetresonanzvorrichtung 10 ein.

Zu einer Steuerung des Grundmagneten 16, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Darstellungseinheit 24, beispielsweise auf zumindest einem Monitor und/oder einem Display, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können. Die Eingabeeinheit 25 kann beispielsweise eine Tastatur und/oder eine Computermaus umfassen. Zudem kann die Eingabeeinheit 25 auch zumindest teilweise einstückig mit der Darstellungseinheit 24 ausgebildet sein, wie beispielsweise bei einer Ausbildung als Touch-Display.

Die dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

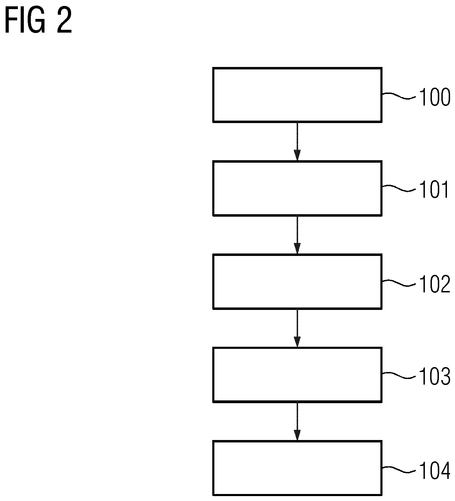

In Fig. 2 ist ein Ablaufplan eines erfindungsgemäßen Verfahrens zu einem Unterstützen eines Benutzers bei einer Einstellung eines Parameterwerts von zumindest einem Messparameter einer Magnetresonanzsequenz für eine Magnetresonanzuntersuchung dargestellt. Zur Ausführung des Verfahrens weist die Magnetresonanzvorrichtung 10 zudem eine Sequenzsteuereinheit 26 auf. Die Sequenzsteuereinheit 26 weist einen Prozessor und eine für die Steuerung des erfindungsgemäßen Verfahrens erforderliche Software und/oder Computerprogramme auf. Die Software und/oder Computerprogramme sind bei einer Ausführung durch den Prozessor der Sequenzsteuereinheit 26 zu einer Steuerung des Verfahrens und/oder zu einer Ausführung von einzelnen Verfahrensschritten des Verfahrens ausgebildet. Die Software und/oder Computerprogramme sind in einer Speichereinheit gespeichert.

Die Speichereinheit kann dabei von der Sequenzsteuereinheit 26 umfasst sein oder auch separat zur Sequenzsteuereinheit 86 ausgebildet sein. Zudem kann die Speichereinheit auch einen separaten Datenträger umfassen. Die Sequenzsteuereinheit 26 ist im vorliegenden Ausführungsbeispiel separat zur Systemsteuereinheit 22 ausgebildet. In einer alternativen Ausbildung der Magnetresonanzvorrichtung 10 kann die Sequenzsteuereinheit 26 auch innerhalb der Systemsteuereinheit 22 integriert sein.

In einem ersten Verfahrensschritt 100 des Verfahrens erfolgt eine manuelle Auswahl zumindest eines Messparameters durch den Benutzer. Die Auswahl des zumindest einen Messparameters erfolgt hierbei mittels der Darstellungseinheit 24 der Benutzerschnittstelle 23 der Magnetresonanzvorrichtung 10. Beispielsweise kann hierbei der Benutzer denjenigen und/oder diejenigen Messparameter auswählen, dessen Parameterwert und/oder deren Parameterwerte er für eine Anpassung an eine medizinische und/oder diagnostische Fragestellung ändern möchte. Der zumindest eine Messparameter kann beispielsweise eine Anzahl an Schichten und/oder eine Schichtdicke und/oder ein FOV und/oder weitere, dem Fachmann als sinnvoll erscheinende Messparameter umfassen. Des Weiteren wird in dem ersten Verfahrensschritt 100 von dem Benutzer ein gewünschter Parameterwert für den zumindest einen ausgewählten Messparameter mittels der Benutzerschnittstelle 23, insbesondere der Eingabeeinheit 25 der Benutzerschnittstelle 23, eingegeben. Dabei weicht bevorzugt der eingegebenen und/oder gewünschte Parameterwert von einem bereits voreingestellten Parameterwert ab.

Der gewünschte und/oder eingegebene Parameterwert kann beispielsweise zu einer Erhöhung oder auch zu einer Verringerung einer Anzahl an Schichten gegenüber einer voreingestellten Anzahl an Schichten führen. Ein weiteres Beispiel für einen eingegeben und/oder gewünschten Parameterwert kann beispielsweise eine Vergrößerung des FOV gegenüber dem voreingestellten FOV umfassen. Zudem sind weitere, dem Fachmann als sinnvoll erscheinende Eingaben für Änderungen des Parameterwerts des zumindest einen ausgewählten Messparameters jederzeit möglich.

In einem weiteren, zweiten Verfahrensschritt 101 erfolgt ein Überprüfen des eingegebenen Parameterwerts des zumindest einen ausgewählten Messparameters. Die Überprüfung erfolgt mittels der Sequenzsteuereinheit 26. Insbesondere erfolgt die Überprüfung automatisch und/oder selbsttätig mittels der Sequenzsteuereinheit 26. Die Überprüfung des eingegebenen Parameterwerts für den zumindest einen ausgewählten Messparameter umfasst eine Inkompatibilitätsprüfung, ob der eingegebene Parameterwert und/oder der gewünschte Parameterwert des zumindest einen ausgewählten Messparameters mit Einstellung von Parameterwerten von weiteren Messparametern der Magnetresonanzsequenz inkompatibel ist. Eine Inkompatibilität zwischen den einzelnen Messparametern, insbesondere den Parameterwerten der einzelnen Messparameter, liegt vor, wenn der eingegebene Parameterwert des zumindest einen ausgewählten Messparameters für eine Ausführung der Magnetresonanzsequenz zu einer Änderung in Parameterwerten von zumindest einem weiteren Messparameter führt.

Sofern der eingegebene Parameterwert des zumindest einen ausgewählten Messparameters mit den Einstellungen von Parameterwerten zumindest eines weiteren Messparameters der Magnetresonanzsequenz inkompatibel ist, erfolgt in einem weiteren, dritten Verfahrensschritt 102 ein Bestimmen von zumindest einer Option für eine Änderung des Parameterwerts des zumindest einen weiteren Parameterwerts. Die Bestimmung der zumindest einen Option für eine Änderung des Parameterwerts des zumindest einen weiteren Messparameters erfolgt mittels der Sequenzsteuereinheit 26. Insbesondere erfolgt die Bestimmung der zumindest einen Option für die Änderung des Parameterwerts des zumindest einen weiteren Messparameters automatisch und/oder selbsttätig mittels der Sequenzsteuereinheit 26. Die Änderung des Parameterwerts des zumindest einen weiteren Messparameters führt zu einer Kompatibilität des eingegebenen Parameterwerts des zumindest einen ausgewählten Messparameters mit den Einstellungen der Parameterwerte der weiteren Messparameter. Neben der Änderung des Parameterwerts für den zumindest einen weiteren Messparameter umfasst die zumindest eine Option auch eine Auswirkung der Änderung des Parameterwerts des zumindest einen weiteren Messparameters auf die Magnetresonanzmessung.

Bevorzugt werden in diesem dritten Verfahrensschritt 102 mehr als eine Option, insbesondere zwei oder mehr Optionen von der Sequenzsteuereinheit 26 bestimmt. Jede der zwei oder mehr Optionen umfasst einen Vorschlag zum Einstellen der Parameterwerte der Messparameter. Insbesondere umfasst eine Option einen Vorschlag zum Einstellen des Parameterwerts des zumindest einen ausgewählten Messparameters mit den eingegebenen und/oder gewünschten Parameterwert und zum Einstellen und/oder Anpassen eines Parameterwerts zumindest eines weiteren Messparameters. Vorzugsweise unterschieden sich die einzelnen Optionen hinsichtlich einer Auswahl der weiteren Messparameter und/oder hinsichtlich der Parameterwerte der weiteren Messparameter. Zudem umfasst eine Option der zwei oder mehr Optionen ein Beibehalten der voreingestellten Parameterwerte aller Messparameter.

In einem weiteren, vierten Verfahrensschritt 103 erfolgt ein Darstellen der zumindest einen Option, insbesondere der zwei oder mehr Optionen für einen Benutzer. Das Darstellen der zumindest einen Option, insbesondere der zwei oder mehr Optionen, erfolgt mittels der Darstellungseinheit 24 der Benutzerschnittstelle 23.

Wird beispielsweise in dem ersten Verfahrensschritt 100 vom Benutzer der Messparameter "Anzahl der Schichten" ausgewählt und der voreingestellte Parameterwert erhöht, beispielsweise von 10 Schichten auf 30 Schichten, erfolgt in dem zweiten Verfahrensschritt 101 die Überprüfung durch die Sequenzsteuereinheit 26. Sofern der eingegebene Parameterwert des zumindest einen Messparameters mit den Einstellungen von Parameterwerten zumindest eines weiteren Messparameters der Magnetresonanzsequenz inkompatibel ist, wird bei der Überprüfung eine Inkompatibilität zwischen der gewünschten und/oder eingegeben Anzahl an Schichten, die vorläufig auf 30 gesetzt ist, und den Einstellungen von Parameterwerten der weiteren Messparameter von der Sequenzsteuereinheit 26 ermittelt. Im dritten Verfahrensschritt 102 wird von der Sequenzsteuereinheit 26 zumindest eine Option, bevorzugt zwei oder mehr Optionen, zur Einstellung der Messparameter ermittelt. Eine erste Option umfasst einen Vorschlag zur Einstellung der Anzahl der Schichten mit der gewünschten Anzahl von 30 und eine damit verbundene Änderung und/oder Anpassung des Parameterwerts eines ersten weiteren Messparameters. Der erste weitere Messparameter umfasst beispielsweise die Echozeit, wobei für die ersten Option eine Erhöhung des Parameterwerts für die Echozeit von beispielsweise 11 auf 15 vorgeschlagen wird. Zudem weist diese erste Option die Information auf, welche Auswirkungen die Änderung und/oder Anpassung der Anzahl der Schichten auf die gewünschte Anzahl der Schichten und die Änderung und/oder Anpassung der Echozeit auf die Magnetresonanzmessung haben würde. Beispielsweise weist die erste Option die Information auf, dass diese Einstellungen eine Erhöhung einer Messzeit für die Magnetresonanzuntersuchung bewirken würden. Dabei kann die Information auch eine genaue Angabe in Sekunden und/oder Minuten, um die sich die Messzeit der Magnetresonanzuntersuchung erhöhen würde, umfassen.

Eine zweite Option umfasst ebenfalls einen Vorschlag zur Einstellung der Anzahl der Schichten mit der gewünschten Anzahl von 30 und eine damit verbundene Änderung und/oder Anpassung des Parameterwerts eines zweiten weiteren Messparameters. Der zweite weitere Messparameter umfasst beispielsweise das FOV, wobei für die zweite Option eine Reduzierung des Parameterwerts für das FOV von beispielsweise 100 % auf 80 % vorgeschlagen wird. Zudem weist diese zweite Option die Information auf, welche Auswirkungen die Änderung und/oder Anpassung der Anzahl der Schichten auf die gewünschte Anzahl der Schichten und die Änderung und/oder Anpassung des FOV auf die Magnetresonanzmessung haben würde. Beispielsweise weist die zweite Option die Information auf, dass diese Einstellungen eine Reduktion des S/N-Verhältnisses für die Magnetresonanzuntersuchung bewirken würden. Dabei kann die Information auch eine genaue Angabe, um wieviel % sich das /N-Verhältnis reduzieren würde, umfassen.

Eine dritte Option kann ebenfalls einen Vorschlag zur Einstellung der Anzahl der Schichten mit der gewünschten Anzahl von 30 und eine damit verbundene Änderung und/oder Anpassung des Parameterwerts eines dritten weiteren Messparameters umfassen. Der dritte weitere Messparameter kann beispielsweise eine Auflösung umfassen, wobei für die dritte Option eine Reduzierung des Parameterwerts für die Auflösung vorgeschlagen wird. Ebenfalls umfasst die dritte Option auch eine Information, welche Auswirkungen die Änderung und/oder Anpassung der Anzahl der Schichten auf die gewünschte Anzahl der Schichten und die Änderung und/oder Anpassung der Auflösung auf die Magnetresonanzmessung haben würde.

Zudem wird eine weitere Option von der Sequenzsteuereinheit 26 generiert, welche ein Beibehalten der voreingestellten und/oder ursprünglichen Parameterwerte aller Messparameter umfasst. Für diese Option wird auch für den ausgewählten Messparameter der voreingestellte und/oder ursprüngliche Parameterwert gewählt. Bei dieser Option würde der vom Benutzer eingegebene und/oder gewünschte Parameterwert auf den ursprünglichen Parameterwert für den zumindest einen Messparameter zurückgesetzt werden, im vorliegenden Ausführungsbeispiel der Parameterwert für die Anzahl der Schichten auf 10 zurückgesetzt werden.

In dem vierten Verfahrensschritt 103 werden alle diese Optionen für den Benutzer mittels der Darstellungseinheit 24 der Benutzerschnittstelle 23 dargestellt.

In einem weiteren, insbesondere optionalen, fünften Verfahrensschritt 104 wird eine der dargestellten Optionen ausgewählt. Die Auswahl erfolgt bevorzugt manuell durch den Benutzer an der Benutzerschnittstelle 23 der Magnetresonanzvorrichtung 10. Nachdem eine Option ausgewählt wurde, werden die Parameterwerte für die Messparameter gemäß der ausgewählten Option eingestellt und/oder angepasst. Die Einstellung und/oder Anpassung der Parameterwerte für die Messparameter erfolgt automatisch und/oder selbsttätig mittels der Sequenzsteuereinheit 26.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einem Unterstützen eines Benutzers bei einer Einstellung eines Parameterwerts von zumindest einem Messparameter einer Magnetresonanzsequenz für eine Magnetresonanzuntersuchung, umfassend die folgenden Verfahrensschritte:
- Manuelle Auswahl zumindest eines Messparameters und Eingabe eines gewünschten Parameterwerts für den zumindest einen Messparameter,
- Überprüfen des eingegebenen Parameterwerts des zumindest einen Messparameters, ob der eingegebene Parameterwert des zumindest einen Messparameters mit Einstellungen von Parameterwerten von weiteren Messparametern der Magnetresonanzsequenz inkompatibel ist,
- sofern der eingegebene Parameterwert des zumindest einen Messparameters mit den Einstellungen von Parameterwerten zumindest eines weiteren Messparameters der Magnetresonanzsequenz inkompatibel ist, Bestimmen von zumindest einer Option für eine Änderung eines Parameterwerts zumindest eines weiteren Messparameters, wobei die Änderung des Parameterwerts des zumindest einen weiteren Messparameters zu einer Kompatibilität des eingegebenen Parameterwerts des zumindest einen Messparameters mit den Einstellungen der Parameterwerte der weiteren Messparameter führt, und
- Darstellen der zumindest einen Option.

2. Verfahren nach Anspruch 1,
**dadurch gekennzeichnet, dass** die zumindest eine Option eine Auswirkung der Änderung des Parameterwerts des zumindest einen weiteren Messparameters auf die Magnetresonanzmessung umfasst.

3. Verfahren nach einem der vorhergehenden Ansprüche,
**dadurch gekennzeichnet, dass** zwei oder mehr Optionen bestimmt und angezeigt werden.

4. Verfahren nach Anspruch 3,
**dadurch gekennzeichnet, dass** eine Option der zwei oder mehr Optionen ein Beibehalten der voreingestellten Parameterwerte aller Messparameter umfasst.

5. Verfahren nach einem der vorhergehenden Ansprüche,
**dadurch gekennzeichnet, dass** eine Option ausgewählt wird und die Parameterwerte der Messparameter gemäß der ausgewählten Option eingestellt werden.

6. Magnetresonanzvorrichtung mit einer Sequenzsteuereinheit und einer Benutzerschnittstelle, wobei die Magnetresonanzvorrichtung zu einem Ausführen eines Verfahrens zu einem Unterstützen eines Benutzers bei einer Einstellung eines Parameterwerts von zumindest einem Messparameter einer Magnetresonanzsequenz für eine Magnetresonanzuntersuchung nach einem der Ansprüche 1 bis 5 ausgebildet ist.

7. Computerprogrammprodukt, welches ein Programm umfasst und direkt in einen Speicher einer programmierbaren Steuereinheit ladbar ist, mit Programmmitteln, um ein Verfahren zu einem Unterstützen eines Benutzers bei einer Einstellung eines Parameterwerts von zumindest einem Messparameter einer Magnetresonanzsequenz für eine Magnetresonanzuntersuchung nach einem der Ansprüche 1 bis 5 auszuführen, wenn das Programm in der Steuereinheit ausgeführt wird.

8. Elektronisch lesbarer Datenträger mit darauf gespeicherten elektronisch lesbaren Steuerinformationen, welche derart ausgestaltet sind, dass sie bei Verwendung des Datenträgers in einer Steuereinheit das Verfahren zu einem Unterstützen eines Benutzers bei einer Einstellung eines Parameterwerts von zumindest einem Messparameter einer Magnetresonanzsequenz für eine Magnetresonanzuntersuchung nach einem der Ansprüche 1 bis 5 durchführen.
